# EUROPÄISCHE PATENTSCHRIFT

(11) **EP 2 272 540 B1**
(45) Veröffentlichungstag und Bekanntmachung des Hinweises auf die Patenterteilung: **06.11.2013**
(21) Anmeldenummer: 10005973.2
(22) Anmeldetag: 10.06.2010
(51) Int. Cl.: A61L 2/22, A61L 2/18, G01N 1/06

(54) **Verfahren und Vorrichtung zur Desinfektion eines Mikrotom-Kyrostaten**
Method and device for disinfecting a microtome cyrostat
Procédé et dispositif de désinfection d'un cryostat à microtome

(30) Priorität: 25.06.2009 DE 102009030624
(43) Veröffentlichungstag der Anmeldung: 12.01.2011
(73) Patentinhaber: Microm International GmbH, 69190 Walldorf (DE)
(72) Erfinder: Teppke, Dieter, 68723 Schwetzingen (DE)
(74) Vertreter: Lasch, Hartmut

(56) Entgegenhaltungen:
- WO-A2-2004/067047
- DE-B3- 10 352 575
- FR-A1- 2 705 587
- US-A- 3 233 965
- US-A1- 2004 238 019

## Beschreibung

Die Erfindung betrifft ein Verfahren zur Desinfektion eines Mikrotom-Kryostaten mit
a) Einbringung eines Desinfektionsmittels in die Kryostatkammer bei unter 0°C, wobei das Desinfektionsmittel bei dieser Temperatur wirksam ist,
b) Einwirkung des Desinfektionsmittels auf die Kryostatkammer,
c) Beseitigung des Desinfektionsmittels durch Niederschlag in einem kälteren Bereich und Abführung von dort.

Die Erfindung betrifft weiterhin eine Vorrichtung zur Desinfektion eines Mikrotom-Kryostaten nach einem Verfahren der vorgenannten Art mit einem Mikrotom, einem Kälteerzeuger, einer Einrichtung zur Bereitstellung und Einbringung eines Desinfektionsmittels in die Kryostatkammer bei einer Temperatur von unter 0°C sowie einer Steuerung.

Da mit Mikrotomen oft Gewebeproben geschnitten werden, die mit Keimen infiziert sind, ist eine Desinfektion zum Schutz von Bedienpersonen geboten. Für solche Desinfektionen wurden folgende Vorschlägen gemacht:
Ein Vorschlag zur Lösung dieses Problems durch die US 2002/0139124 A1 besteht darin, eine Desinfektion mit Ozon vorzunehmen. Dies hat jedoch den Nachteil, daß Ozon sehr stark korrosiv wirkt und daher Oberflächen durch Oxidation beschädigt werden. Ein weiterer Nachteil besteht darin, daß Ozonreste schwer entfernbar, giftig und stark brandfördernd sind, teilweise aber auch schon während der Desinfektionsphase aus dem Gerät austreten und dann Bedienpersonen gefährden können.

In dem Firmenprospekt "AS 600 Cryotome" der Firma ANGLIA SCIENTIFIC besteht ein weiterer Vorschlag darin, eine Dekontamination mit UV-Strahlen vorzunehmen. Dies hat jedoch den Nachteil, daß die UV-Strahlen nicht in Schattenbereiche gelangen. Außerdem ist auch die Eindringtiefe der UV-Strahlen in Schnittabfälle oder Probenreste und in mikroskopisch kleine Vertiefungen von Metalloberflächen nicht gegeben, so daß diese Dekontamination unbefriedigend ist.

Von der DE 103 03 989 B4 wird ein Verfahren und eine Vorrichtung vorgeschlagen, bei denen ein dampfförmiges Desinfektionsmittel in eine Kryostatkammer eingeleitet wird, welche zuvor aufgewärmt wurde. Nach einer Einwirkzeit wird das Desinfektionsmittel dadurch entfernt, daß zwischen der Kryostatkammer und einem Kälteerzeuger eine Temperaturdifferenz erzeugt wird, wodurch das Mikrotom, welches vorzugsweise noch geheizt wird, trocknet und sich das gesamte Desinfektionsmittel an dem wieder in Betrieb gesetzten Kälteerzeuger niederschlägt. Von dort wird es dann durch erneute Erwärmung des Kälteerzeugers abgetaut und abgeführt. Der Kälteerzeuger wird dann wieder in Betrieb genommen, damit die Kryostatkammer ihre Betriebstemperatur wieder erreicht. Diese Vorgehensweise war zwar schon wesentlich schneller als eine Austrocknung durch einfaches Offenstehenlassen des Kryostaten nach einer Desinfektion, bis die Kryostatkammer mit Mikrotom getrocknet ist. Da sich die Zeitdauer aus einer Entfrostungs- und Aufwärmzeit, einer Bereitstellungs- und Einwirkzeit des Desinfektionsmittels sowie der Zeit für die Entfernung des Desinfektionsmittels und der erneuten Kühlung zusammensetzt, werden jedoch immer noch mindestens zwei Stunden bis zur Wiederinbetriebnahme des Mikrotoms benötigt. Damit ist eine Desinfektion entweder nur am Ende eines Arbeitstages möglich oder, falls wegen starker Infektionsgefahr eine öftere Desinfektion unverzichtbar ist, führt dies zu einer wesentlichen Einschränkung der Einsatzfähigkeit des Mikrotom-Kryostaten.

Die FR 2 705 587 lehrt die Desinfektion eines Mikrotom-Kryostaten durch Einbringung eines Desinfektionsmittels in Dampfform mit einer Einwirkzeit von einer Stunde bei mindestens 60°C, einer Austrocknung durch Öffnung der Türe und danach einer erneuten Kühlung der Kryostatkammer auf Betriebstemperatur. Die Betriebsunterbrechung ist damit mindestens genauso lange wie bei dem von der DE 103 03 989 B4 vorgeschlagenen Mikrotom-Kryostaten.

Die US 2004/238019 A1 lehrt einen Mikrotom-Kryostaten und ein Arbeitsverfahren der eingangs genannten Art zur Desinfektion des Mikrotom-Kryostaten. Dabei wird gelehrt, daß eine Sprühdüse die Desinfektionsflüssigkeit bei unter 0°C in die Kryostatkammer sprüht. Von einer reifartigen Ablagerung offenbart diese Schrift nichts, da sie jedoch von einer Entzündbarkeit des Desinfektionsmittels bei über 0°C ausgeht, kann davon ausgegangen werden, daß das verwendete, aber nicht näher angegebene Desinfektionsmittel auch unter 0°C noch flüssig ist. Weiterhin geht diese Schrift davon aus, daß vor jeder Desinfektion eine vorhergehende Abtauung erforderlich ist, damit das Eis, insbesondere das Eis auf den Kühlschlangen abgetaut werden kann, um diese desinfizieren zu können. Letzteres bedeutet, daß es sich bei dem Eis um gefrorene Luftfeuchtigkeit handelt und nicht um gefrorenes Desinfektionsmittel, das auch als Eis noch eine Desinfektionswirkung entfaltet.

Wegen des Abtauens vor jeder Desinfektion wird auch bei diesem Mikrotom-Kryostaten viel Zeit für einen Desinfektionsvorgang benötigt. Deshalb empfiehlt diese Schrift, die Desinfektion außerhalb der üblichen Betriebszeiten vorzunehmen.

Der Erfindung liegt daher die Aufgabe zugrunde, ein Verfahren und eine Vorrichtung der eingangs genannten Art derart auszubilden, daß der Mikrotom-Kryostat ohne längere Betriebsunterbrechung mehrmals täglich desinfiziert werden kann.

Die Aufgabe wird erfindungsgemäß durch folgende Ausgestaltung der eingangs genannten Verfahrensschritte gelöst:
a) Das Desinfektionsmittel wird in nebelartiger Form eingebracht,
b) das Desinfektionsmittel wirkt als reifartige Ablagerung in der Kryostatkammer,
c) die sich durch Sublimation verlagerte reifartige Ablagerung in einen kälteren Bereich wird durch sporadische Entfrostungsphasen dieses Bereichs verflüssigt und dadurch abgeführt.

Die Aufgabe wird durch eine Vorrichtung der eingangs genannten Art gelöst, die dadurch gekennzeichnet ist, daß die Steuerung derart ausgebildet ist, daß sie eine Abtauung des sich am Kälteerzeuger durch Sublimation niedergeschlagenen und angesammelten Desinfektionsmittels veranlaßt, bevor diese Schicht eine Stärke aufweist, welche die Arbeitsweise des Kälteerzeugers beeinträchtigen kann, und daß eine Abführeinrichtung das getaute Desinfektionsmittel aus der Kryostatkammer ableitet.

Der Erfindung liegt die Erkenntnis zugrunde, daß einige der bekannten Desinfektionsmittel auch dann desinfizierend wirken, wenn sie durch Einbringung in nebelartiger Form in eine Kryostatkammer mit unter 0° C dort eine reifartige Ablagerung bilden. Dies macht sich die Erfindung zunutze, um die Desinfektion zu bewirken.

Des weiteren macht sich die Erfindung zunutze, daß sich eine solche reifartige Ablagerung durch Sublimation auch bei einer Temperatur von unter 0° C wieder auflöst, wenn es einen noch kälteren Bereich gibt. Durch die Niederschlagung des Desinfektionsmittels in dem noch kälteren Bereich wird erreicht, daß der Sättigungssublimationsdruck in der Kryostatkammer nicht erreicht wird und daher die Sublimation des reifartig abgelagerten Desinfektionsmittels so lange weitergeht, bis sich alles in dem kälteren Bereich niedergeschlagen hat. Dieser kältere Bereich ist zweckmäßigerweise der Kühler des Mikrotom-Kryostaten, da dieser ohnehin immer dann der kälteste Bereich der Kryostatkammer ist, wenn er in Betrieb ist.

Schließlich wurde noch erkannt, daß die Desinfektionswirkung der reifartig abgelagerten Schicht wesentlich effektiver wirkt als ein Kondensat von Desinfektionsmittel, das sich bei über 0° C in Tröpfchenform niederschlägt. Dabei stellte sich heraus, daß der Desinfektionsmittelbedarf ungefähr um den Faktor 10 reduziert werden konnte. Diese Reduktion des eingesetzten Desinfektionsmittels führte aber wiederum dazu, daß das im kälteren Bereich, wie dem Kühler, niedergeschlagene Desinfektionsmittel auch nicht nach jeder Desinfektion wieder entfernt und abgeführt werden muß, da die dort entstehende Desinfektionsmittelschicht wegen der erheblich geringeren eingesetzten Desinfektionsmittelmenge nach einem Desinfektionsvorgang nur ungefähr ein Zehntel dessen beträgt, als beim oben genannten Verfahren nach dem Stand der Technik. Dies ermöglicht es, den kälteren Bereich, also den Kühler, nur sporadisch abzutauen, da sich dort erst nach mehreren Desinfektionsvorgängen eine Schicht gebildet hat, die entfernt werden sollte, um die Arbeitsweise des Kälteerzeugers nicht zu beeinträchtigen.

Somit spart die Erfindung für die Durchführung der Desinfektion eine Erwärmung der Kryostatkammer vor Einbringung des Desinfektionsmittels genauso ein, wie die zeitaufwendige Entfernung des Desinfektionsmittels aus der Kryostatkammer nach jeder einzelnen Desinfektion. Letztere bestand beim oben genannten Stand der Technik in einer Abkühlung des Kühlers unter 0° C, damit sich das Desinfektionsmittel dort niederschlägt, einem anschließenden Abtauen des Kühlers, damit das Desinfektionsmittel abgeleitet werden kann, und nachfolgend wieder eine Kälteerzeugung, damit die Kryostatkammer wieder ihre Betriebstemperatur erreicht.

Bei der Erfindung kann dagegen die Kryostatkammer bei ihrer üblichen Betriebstemperatur bleiben, wenn das Desinfektionsmittel eingebracht wird. Diese Temperatur liegt je nach den zu verarbeitenden Proben zwischen -10° C und -35° C, üblicherweise bei ca. -20° C. Da bei der üblichen Betriebstemperatur der Kühler ohnehin kälter ist, sublimiert der reifartige Desinfektionsmittelniederschlag zum Kühler, wobei es reicht, wenn dieser wenige ° C kälter ist als Mikrotom und Kryostatkammer. Das Mikrotom kann dann wieder in Betreib genommen werden, wenn die Schicht auf dem Mikrotommesser größtenteils entfernt ist. Die Sublimation des in anderen Teilen der Kryostatkammer niedergeschlagenen Desinfektionsmittels kann dabei unvollständig sein, sie vollendet sich während der Mikrotom-Kryostat schon wieder in Betrieb ist und die Abführung des am Kälteerzeugers niedergeschlagenen Desinfektionsmittels kann dann später durch sporadische Entfrostungsphasen des Kälteerzeugers erfolgen.

Für die sporadische Auftauung und Abführung des im kälteren Bereich (Kühler) niedergeschlagenen Desinfektionsmittels reicht es völlig aus, wenn dies nach ca. sechs bis zehn Desinfektionsvorgängen erfolgt, so daß dieser Vorgang zweckmäßigerweise an das Ende eines Arbeitstages gelegt werden kann, um möglichst keinen Verlust an Einsatzzeit für den Mikrotom-Kryostaten herbeizuführen.

Zweckmäßige Ausgestaltungen des erfindungsgemäßen Verfahrens sind den Unteransprüchen zu entnehmen.

Zur schnellen Durchführung der Desinfektion des Mikrotom-Kryostaten ist vorgesehen, daß das Desinfektionsmittel bei der üblichen Betriebstemperatur der Kryostatkammer in diese eingeführt wird, dadurch entfällt die Entfrostungs- und Aufwärmzeit des oben beschriebenen Standes der Technik völlig. Bei der Durchführung der Desinfektion wird die übliche Kühlung der Kryostatkammer nicht unterbrochen, sondern für den Sublimationseffekt genutzt, da die Temperaturdifferenz zwischen der auf Betriebstemperatur gekühlten Kryostatkammer und dem diese kühlenden Kälteerzeuger so groß ist, daß der oben genannte Sublimationseffekt erreicht wird.

Die Einbringung des nebelartigen Desinfektionsmittels dauert vorzugsweise ca. drei Minuten, da sich herausgestellt hat, daß dies ausreichend ist, um die vorhandenen Keime abzutöten. Ausreichend insofern, als die erforderliche Dicke und Vollständigkeit der reifartigen Ablagerung dadurch erzielt wird, und diese Ablagerung auch die notwendige Zeitdauer auf den zu desinfizierenden Flächen verweilt. Dabei kommt zur Einbringzeit für das Desinfektionsmittel noch die Zeit hinzu, die erforderlich ist, damit das Mikrotommesser durch die Sublimation wieder weitestgehend freigegeben ist. Diese Zeit für die Sublimation wird allerdings für die Desinfektionswirkung nur teilweise benötigt, so daß diese Zeit gemäß Weiterbildungen der Erfindung verkürzt werden kann.

Es hat sich herausgestellt, daß es ausreicht, wenn beim erfindungsgemäßen Verfahren 15 bis 25 ml Desinfektionsmittel in die Kryostatkammer eingebracht werden. Dies ist, wie oben bereits erwähnt, wesentlich weniger als beim oben genannten Stand der Technik, bei dem ca. 200 ml des Desinfektionsmittels erforderlich waren. Gerade diese geringere Menge macht es auch möglich, den Sublimationseffekt zu nutzen, auf eine stärkere Erwärmung zur Erzielung einer Verdunstung zu verzichten und trotzdem noch Zeit einzusparen.

Die reifartige Ablagerung des Desinfektionsmittels wirkt um so effektiver, je kleiner die Partikelgrößen der eingebrachten nebelartigen Form des Desinfektionsmittels sind. Um eine solche möglichst feine Partikelgröße zu erreichen, ist es zweckmäßig, wenn die Vernebelung des Desinfektionsmittels vor der Einführung in die Kryostatkammer mittels Ultraschall erfolgt.

Wie schon erwähnt, dient als kälterer Bereich zweckmäßigerweise der Kälteerzeuger des Mikrotom-Kryostaten. Dieser wird zum Abtauen und Abführen des dort abgelagerten Desinfektionsmittels in derart sporadischen Zeitabständen erwärmt, daß sich dort keine die Funktionsfähigkeit des Kälteerzeugers beeinträchtigende gefrorene Desinfektionsmittelschicht bildet, sondern diese vor Erreichen einer diesbezüglich kritischen Stärke beseitigt wird. Dabei reicht die Größenordnung von einer Abtauung nach sechs bis zehn Desinfektionsvorgängen im allgemeinen völlig aus. Auf diese Weise kann das Abtauen und Abführen einmal täglich vorgenommen werden.

Die einzelnen Desinfektionsmitteleinbringungen richten sich sowohl nach der Anzahl der zu verarbeitenden Proben, als auch nach dem Ausmaß der durch diese hervorgerufenen Infektionsgefahr. Darum ist es zweckmäßig, wenn die Ingangsetzung der Desinfektionsmitteleinbringung nach dem jeweiligen Desinfizierungsbedarf erfolgt, wobei dies zweckmäßigerweise von der Bedienperson veranlaßt wird, die diesen Bedarf einschätzen kann. Um jedoch auf jeden Fall ein Minimum an Desinfizierung zu gewährleisten, ist es zweckmäßig, wenn mindestens eine Desinfektionsmitteleinbringung täglich automatisch erfolgt.

Nach dem jeweiligen Desinfektionsvorgang kann das Mikrotom dann wieder in Betrieb genommen werden, wenn die reifartige Desinfektionsmittelschicht auf dem Mikrotommesser größtenteils entfernt ist. Eine völlige Entfernung ist also nicht immer notwendig. Ein Verbleiben einer Desinfektionsmittelschicht auf den übrigen Flächen der Kryostatkammer ist völlig unschädlich, diese kann auch im Laufe der nächsten Schneidevorgänge zum Kälteerzeuger sublimieren.

Zweckmäßigerweise kann zur schnelleren Wiederinbetriebnahme des Mikrotoms nach einer Desinfektion die Beseitigung der reifartigen Ablagerung des Desinfektionsmittels am Mikrotommesser nach der notwendigen Einwirkzeit mechanisch erfolgen. Dies ist sowohl manuell mittels eines Pinsels als auch mittels einer entsprechenden mechanischen Vorrichtung möglich. Eine andere Möglichkeit besteht darin, daß die Entfernung der Desinfektionsmittelschicht vom Mikrotommesser nach der notwendigen Einwirkzeit durch eine Erwärmung desselben beschleunigt wird. Eine solche erhöhte Temperatur muß dabei gar nicht über 0° C gehen, bereits eine Anhebung um einige Grad Celsius beschleunigt wesentlich den Sublimationseffekt. Da das Mikrotommesser das Teil des Mikrotom-Kryostaten ist, das für die Funktionsfähigkeit auf jeden Fall die entsprechend tiefe Temperatur aufweisen muß, ist es zweckmäßig, wenn nach einer solchen Erwärmung ein Wiedererreichen der tiefen Betriebstemperatur des Mikrotommessers durch eine Kühlung desselben beschleunigt wird. Auf diese Weise wird die Arbeitsunterbrechung durch die Desinfektion nochmals erheblich reduziert.

Für die Desinfektion bei den tiefen Betriebstemperaturen hat sich die Verwendung einer Wasserstoffperoxidlösung in Wasser als geeignetes Desinfektionsmittel erwiesen. Dabei ist die Desinfektionswirkung bei solchen tiefen Temperaturen dann besonders effektiv, wenn diese Wasserstoffperoxidlösung einen geringen Anteil Silber enthält.

Entsprechend der oben genannten Weiterbildung des erfindungsgemäßen Verfahrens sieht auch eine Weiterbildung der erfindungsgemäßen Vorrichtung vor, daß die Vernebelungsvorrichtung mit einem Ultraschallgenerator ausgestattet ist, um mittels Ultraschall eine Feinheit des Nebels zu erzeugen, die besonders effektiv ist. Um alle Teile der Kryostatkammer und dabei aber besonders effektiv das Mikrotom mit dem Desinfektionsmittel zu erreichen, ist es zweckmäßig, wenn die Einrichtung zur Bereitstellung und Einbringung eines Desinfektionsmittels einen Ventilator aufweist, der die Kryostatkammer von oben mit dem vernebelten Desinfektionsmittel beaufschlagt.

Da auch das sporadische Abtauen des Kälteerzeugers nicht zuviel Zeit in Anspruch nehmen sollte, ist es zweckmäßig, wenn dieser zur Bewirkung eines schnellen Abtauens eine Heizeinrichtung aufweist. Dann ist es auch zweckmäßig, wenn der Kälteerzeuger eine Auffangvorrichtung zum Auffangen des abgetauten Desinfektionsmittels aufweist, um dieses mittels der Abführvorrichtung aus der Kryostatkammer herauszuführen.

Vorzugsweise ist eine Eingabevorrichtung zur manuellen Ingangsetzung der Desinfektionsmitteleinbringung vorgesehen, damit die Bedienperson diese nach Bedarf betätigen kann, insbesondere dann öfter, wenn die Infektionsgefahr besonders hoch ist. Auf jeden Fall sollte jedoch die Steuerung derart ausgebildet sein, daß mindestens eine Desinfektionsmitteleinbringung täglich bewirkt wird. Dies kann man als übliche Mindestdesinfektion betrachten, die auch dann vorgenommen werden sollte, wenn Proben verarbeitet wurden, die bezüglich Infektionsgefahr unproblematisch sind.

Weiterhin sollte die Steuerung derart ausgebildet sein, daß sie das Abtauen des Kälteerzeugers einmal täglich bewirkt. Dies schließt natürlich nicht aus, daß das Abtauen öfter vorgenommen wird, gewährleistet aber unter normalen Betriebsbedingungen, daß die Schicht gefrorenen Desinfektionsmittels auf dem Kälteerzeuger auf keinen Fall zu groß wird. Außerdem kann auf diese Weise das Ende des Arbeitstages ausgenutzt werden, um keine Unterbrechung der Arbeiten durch ein Abtauen im Laufe eines Arbeitstages in Kauf nehmen zu müssen. Selbstverständlich könnte natürlich auch ein Sensor vorgesehen sein, der die Dicke der Schicht gefrorenen Desinfektionsmittels auf dem Kälteerzeuger erfaßt und anzeigt und/oder das Abtauen veranlaßt.

Die Arbeitsunterbrechungen durch Desinfektionsvorgänge können durch eine Einrichtung zur beschleunigten Entfernung der reifartigen Ablagerung eines Desinfektionsmittels am Mikrotommesser nach der notwendigen Einwirkzeit, die wenige Minuten betragen kann, verkürzt werden. Dabei kann es sich um eine Einrichtung zur mechanischen Entfernung der reifartigen Ablagerungen handeln, oder es kann auch vorgesehen sein, daß diese Einrichtung eine Heizung für das Mikrotommesser ist. Eine solche kann auch mit einer Kühleinrichtung für das Mikrotommesser verknüpft sein, um nach der Entfernung der reifartigen Ablagerungen eine möglichst schnelle Kühlung des Mikrotommessers auf seine Betriebstemperatur zu bewirken. Für eine Bewirkung von Heizung und Kühlung durch ein einziges Element bietet sich dabei ein Peltierelement an.

Die Vorrichtung ist zweckmäßigerweise für die Handhabung einer Wasserstoffperoxidlösung als Desinfektionsmittel optimiert, insbesondere für eine solche, die einen geringen Silberanteil aufweist. Diese Optimierung beinhaltet sowohl die Einwirkungszeit zur sicheren Desinfektion als auch die erforderliche Zeit für die Sublimation einer solchen Wasserstoffperoxidlösung. Entsprechend müssen also die Zeiten und Temperaturen an eine solche Wasserstoffperoxidlösung angepaßt sein.

Selbstverständlich können die nur zum Verfahren offenbarten Weiterbildungen in entsprechender Weise auch bei der Vorrichtung vorgesehen sein, ebenso auch umgekehrt. Weitere Ausgestaltungen sind möglich, beispielsweise auch mehrere Kälteerzeugungseinrichtungen, um beispielsweise die Temperatur von Probe und Messer für das Schneiden bestimmter Proben weiter abzusenken, wobei dann natürlich Vorsorge dafür getroffen werden muß, daß die Sublimation nur zu einem noch kälteren Bereich (Kälteerzeuger) erfolgt.

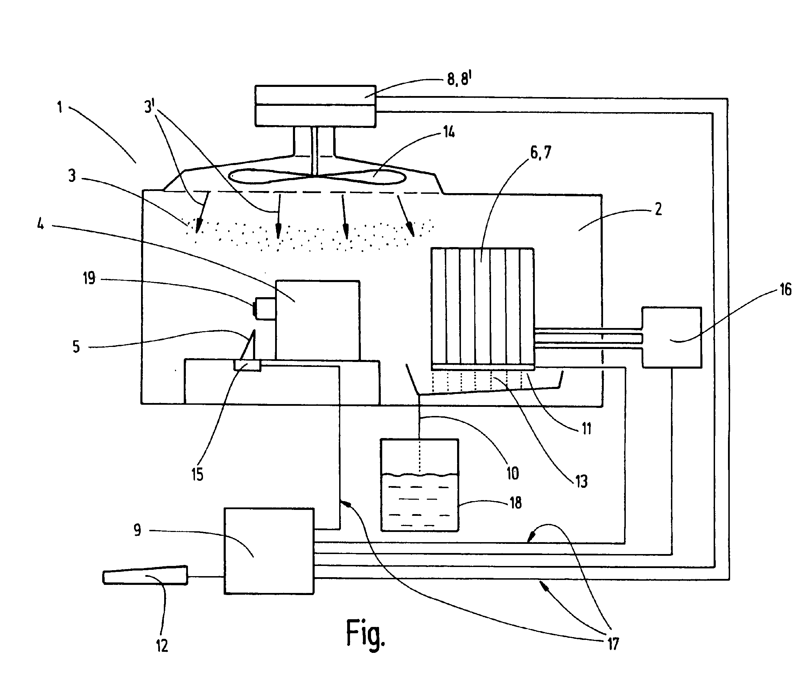

Die Erfindung wird nachfolgend anhand eines in der Zeichnung dargestellten Ausführungsbeispiels der Vorrichtung erläutert. Dargestellt ist ein Mikrotom-Kryostat 1 mit einer Kryostatkammer 2, in dem sich das Mikrotom 4 befindet, das mittels eines Mikrotommessers 5 gefrorene Proben 19 schneidet. Die Kryostatkammer 2 verfügt über einen Kälteerzeuger 7, welcher als kälterer Bereich 6 im Sinne des erfindungsgemäßen Verfahrens dient. Der Kälteerzeuger 7 wird mittels einer Kühlmittelversorgung 16 betrieben, die sich außerhalb der Kryostatkammer 2 befindet.

Zur Desinfektion des Mikrotom-Kryostaten 1 dient eine Einrichtung 8 zur Bereitstellung und Einbringung eines Desinfektionsmittels 3, wie durch die Pfeile 3' angezeigt ist. Dabei sorgt ein Ventilator 14 für eine gleichmäßige Beaufschlagung der Kryostatkammer 2 mit dem Desinfektionsmittel 3. Um eine möglichst homogene und feinkörnige Reifschicht aus Desinfektionsmittel 3 in der Kryostatkammer 2 zu erzielen, ist die Einrichtung 8 mit einer Vernebelungseinrichtung 8' ausgestattet, die vorzugsweise mit Ultraschall arbeitet.

Unter dem Kälteerzeuger 7 befindet sich eine Auffangvorrichtung 13, in die getautes Desinfektionsmittel 3 hineinläuft, wenn der Kälteerzeuger 7 entfrostet wird. Von dieser Auffangvorrichtung 13 gelangt das Kühlmittel 3 in eine Abführvorrichtung 10, die das Desinfektionsmittel 3 aus der Kryostatkammer 2 herausleitet, beispielsweise in einen Auffangbehälter 18 für die Abtauflüssigkeit.

Der Bedienung des Geräts und der Durchführung des Verfahrens dienen eine Steuerung 9 sowie eine Eingabevorrichtung 12. Die Steuerung 9 weist Verbindungsleitungen 17 auf, die zur Einrichtung 8 zur Bereitstellung und Einbringung eines Desinfektionsmittels 3 führen, dort beispielsweise zur Vernebelungsvorrichtung 8', sowie zum Ventilator 14. Weiterhin geht eine Verbindungsleitung 17 zur Kühlmittelversorgung 16 des Kälteerzeugers 7, sowie zu einer Heizeinrichtung 11 für den Kälteerzeuger 7. Eine weitere Verbindungsleitung 17 geht noch zu einem Peltierelement 15, das am Mikrotommesser 5 angeordnet ist.

Die Funktionsweise der Vorrichtung ist folgende:
Wenn nach dem Schneiden von Proben 19 eine Desinfektion zur Abtötung von Keimen erforderlich ist, wird die Einrichtung 8 mit der Vernebelungsvorrichtung 8' und dem Ventilator 14 in Gang gesetzt, um in Richtung der Pfeile 3' das Desinfektionsmittel 3 in die kalte Kryostatkammer 2 einzuführen. Die Kryostatkammer 2 befindet sich dabei in der üblichen Betriebstemperatur, die zwischen -10 und -35° C liegen kann, in der Regel jedoch bei ca. -20° C liegt. Das nebelartig fein verteilte Desinfektionsmittel 3 setzt sich dann in der gesamten Kryostatkammer 2 nieder. Dabei reicht es aus, wenn die Einbringung des nebelartigen Desinfektionsmittels 3 ca. drei Minuten dauert und in dieser Zeit 15 bis 25 ml Desinfektionsmittel 3 in die Kryostatkammer 2 eingebracht werden. Da der Kälteerzeuger 7 weiterarbeitet, bildet er einen kälteren Bereich 6, durch den eine Sublimation des in der Kryostatkammer 2 niedergeschlagenen Desinfektionsmittels 3 in Gang gesetzt wird. Dadurch sublimiert die reifartige Ablagerung von Desinfektionsmittel 3 in der Kryostatkammer 2 und setzt sich an dem Kälteerzeuger 7 nieder, der dazu lediglich wenige Grad kälter sein muß. Wenn auf diese Weise zumindest das Mikrotommesser 5 wieder frei oder weitgehend frei von der reifartigen Ablagerung des Desinfektionsmittels 3 ist, kann die nächste Probe 19 geschnitten werden.

Dabei kann die Zeitdauer bis zum Freiwerden des Mikrotommessers 5 von der reifartigen Ablagerung auch dadurch verkürzt werden, daß diese durch eine entsprechende Einrichtung oder einfach mit einem Pinsel von der reifartigen Ablagerung befreit wird. Dadurch ist es möglich, nach wenigen Minuten Einwirkzeit des Desinfektionsmittels die nächsten Schnitte wieder vornehmen zu können. Das Mikrotommesser 5 kann jedoch auch mit einer Heizeinrichtung ausgestattet werden, um die Sublimation an dieser Stelle zu beschleunigen. Bei dem dargestellten Ausführungsbeispiel handelt es sich um ein Peltierelement 15, mit dem für die Beschleunigung der Sublimation - oder um gezielt örtlich begrenzt auf das Mikrotommesser 5 ein Abtauen und eine Verdunstung zu bewirken - kurz geheizt werden kann, und danach gekühlt, um das Mikrotommesser 5 sehr schnell wieder auf seine Betriebstemperatur für den nächsten Schnitt zu bringen.

Da nach dem erfindungsgemäßen Verfahren relativ wenig Desinfektionsmittel 3 für eine Desinfektion benötigt wird, ist es nicht erforderlich, den Kälteerzeuger 7 nach jedem Desinfektionsvorgang abzutauen. Es ist nicht einmal erforderlich, daß der gesamte reifartige Belag der Kryostatkammer 2 vor der Fortsetzung der Schneidarbeiten zu dem Kälteerzeuger 7 sublimiert ist, dazu reicht völlig aus, daß das Mikrotommesser 5 weitestgehend frei von dem reifartigen Belag ist, da sich die Sublimation von den übrigen Flächen der Kryostatkammer 2 zum Kälteerzeuger 7 während der weiteren Schneidearbeiten fortsetzt und vollendet. Erst nach mehreren Desinfektionsvorgängen, beispielsweise nach sechs bis zehn, sollte der Kälteerzeuger 7 abgetaut werden, damit die dort gebildete Schicht gefrorenen Desinfektionsmittels 3 die Kälteerzeugung nicht behindert. Sechs bis zehn Desinfektionsvorgänge finden bei durchschnittlichen Proben 19 ungefähr an einem Tag statt, so daß das Abtauen des Kälteerzeugers 7 in der Regel am Ende des Arbeitstages vorgenommen werden kann. Dazu dient eine Heizeinrichtung 11, die die Temperatur des abgeschalteten Kälteerzeuger 7 über 0° C anhebt und dadurch das Desinfektionsmittel 3, das sich auf dem Kälteerzeuger 7 angesammelt hat, abtaut. Das Desinfektionsmittel 3 läuft dann in die Auffangvorrichtung 13 und wird mittels der Abführeinrichtung 10 in einen Auffangbehälter 18 für die Abtauflüssigkeit geleitet, der sich außerhalb der Kryostatkammer 2 befindet. Danach kann der Mikrotom-Kryostat 1 wieder gekühlt und in Betrieb genommen werden.

Die Darstellung zeigt lediglich eine mögliche Ausgestaltung eines nach der Erfindung arbeitenden Mikrotom-Kryostaten 1. Variationen sind insbesondere in der Anordnung möglich, die ohnehin nur symbolisch dargestellt ist.

### Bezugszeichenliste

- 1: Mikrotom-Kryostat
- 2: Kryostatkammer
- 3: Desinfektionsmittel
- 4: Mikrotom
- 5: Mikrotommesser
- 6: kälterer Bereich
- 7: Kälteerzeuger
- 8: Einrichtung zur Bereitstellung und Einbringung eines Desinfektionsmittels
- 8': Vernebelungsvorrichtung
- 9: Steuerung
- 10: Abführeinrichtung
- 11: Heizeinrichtung
- 12: Eingabevorrichtung
- 13: Auffangvorrichtung
- 14: Ventilator
- 15: Peltierelement
- 16: Kühlmittelversorgung des Kälteerzeugers
- 17: Verbindungsleitungen der Steuerung
- 18: Auffangbehälter für Abtauflüssigkeit
- 19: Probe

## Patentansprüche

1. Verfahren zur Desinfektion eines Mikrotom-Kryostaten (1) mit
a) Einbringung eines Desinfektionsmittels (3) in die Kryostatkammer (2) bei unter 0°C, wobei das Desinfektionsmittel (3) bei dieser Temperatur wirksam ist,
b) Einwirkung des Desinfektionsmittels (3) auf die Kryostatkammer (2),
c) Beseitigung des Desinfektionsmittels (3) durch Niederschlag in einem kälteren Bereich (6) und Abführung von dort,
**gekennzeichnet durch** folgende Ausgestaltung dieser Verfahrensschritte:
a) Das Desinfektionsmittel (3) wird in nebelartiger Form eingebracht,
b) das Desinfektionsmittel (3) wirkt als reifartige Ablagerung in der Kryostatkammer (2),
c) die sich **durch** Sublimation verlagerte reifartige Ablagerung in einen kälteren Bereich (6) wird **durch** sporadische Entfrostungsphasen dieses Bereichs (6) verflüssigt und **dadurch** abgeführt.

2. Verfahren nach Anspruch 1,
**dadurch gekennzeichnet,**
**daß** das Desinfektionsmittel (3) bei der üblichen Betriebstemperatur der Kryostatkammer (2) in diese eingeführt wird.

3. Verfahren nach Anspruch 1 oder 2,
**dadurch gekennzeichnet,**
**daß** die Einbringung des nebelartigen Desinfektionsmittels (3) 3 Minuten dauert.

4. Verfahren nach Anspruch 1, 2 oder 3,
**dadurch gekennzeichnet,**
**daß** 15 bis 25 ml Desinfektionsmittel (3) in die Kryostatkammer (2) eingebracht werden.

5. Verfahren nach einem der Ansprüche 1 bis 4,
**dadurch gekennzeichnet,**
**daß** die Vernebelung des Desinfektionsmittels (3) vor der Einführung (3') in die Kryostatkammer (2) mittels Ultraschall erfolgt.

6. Verfahren nach einem der Ansprüche 1 bis 5,
**dadurch gekennzeichnet,**
**daß** als kälterer Bereich (6) ein Kälteerzeuger (7) dient, der zum Abtauen und Abführen des dort abgelagerten Desinfektionsmittels (3) in derart sporadischen Zeitabständen erwärmt wird, daß sich dort keine gefrorene Desinfektionsmittelschicht in einer solchen Stärke bilden kann, daß die Funktionsfähigkeit des Kälteerzeugers (7) beeinträchtigt wird.

7. Verfahren nach Anspruch 6,
**dadurch gekennzeichnet,**
**daß** das Abtauen und Abführen einmal täglich erfolgt.

8. Verfahren nach einem der Ansprüche 1 bis 7,
**dadurch gekennzeichnet,**
**daß** die Ingangsetzung der Desinfektionsmitteleinführung (3') nach dem jeweiligen Desinfizierungsbedarf erfolgt.

9. Verfahren nach Anspruch 8,
**dadurch gekennzeichnet,**
**daß** mindestens eine Desinfektionsmitteleinführung (3') täglich automatisch erfolgt.

10. Verfahren nach einem der Ansprüche 1 bis 9,
**dadurch gekennzeichnet,**
**daß** das Mikrotom (4) wieder in Betrieb genommen wird, wenn die reifartige Desinfektionsmittelschicht auf dem Mikrotommesser (5) entfernt ist.

11. Verfahren nach Anspruch 10,
**dadurch gekennzeichnet,**
**daß** zur schnelleren Wiederinbetriebnahme des Mikrotoms (4) nach einer Desinfektion die Beseitigung der reifartigen Ablagerung des Desinfektionsmittels (3) am Mikrotommesser (5) nach der notwendigen Einwirkzeit mechanisch erfolgt.

12. Verfahren nach Anspruch 10,
**dadurch gekennzeichnet,**
**daß** die Entfernung der Desinfektionsmittelschicht nach der notwendigen Einwirkzeit vom Mikrotommesser (5) durch eine Erwärmung desselben beschleunigt wird.

13. Verfahren nach Anspruch 12,
**dadurch gekennzeichnet,**
**daß** ein Wiedererreichen der Betriebstemperatur des Mikrotommessers (5) durch eine Kühlung desselben beschleunigt wird.

14. Verfahren nach einem der Ansprüche 1 bis 13,
**dadurch gekennzeichnet,**
**daß** als Desinfektionsmittel (3) eine Wasserstoffperoxidlösung verwendet wird.

15. Verfahren nach Anspruch 14,
**dadurch gekennzeichnet,**
**daß** diese einen Anteil Silber enthält.

16. Vorrichtung zur Desinfektion eines Mikrotom-Kryostaten (1) nach einem Verfahren gemäß einem der vorgenannten Ansprüche mit einem Mikrotom (4), einem Kälteerzeuger (7), einer Einrichtung (8) zur Bereitstellung und Einbringung eines Desinfektionsmittels (3) in die Kryostatkammer (2) sowie einer Steuerung (9),
wobei die Einrichtung (8) zur Bereitstellung und Einbringung eines Desinfektionsmittels (3), eine derart ausgestaltete Vernebelungsvorrichtung (8') ist, daß durch die Feinheit und die Art der Einbringung des vernebelten Desinfektionsmittels (3) in die Kryostatkammer (2) eine reifartige Ablagerung des Desinfektionsmittels (3) in der Kryostatkammer (2) bewirkbar ist, daß die Steuerung (9) derart ausgebildet ist, daß sie eine Abtauung des sich am Kälteerzeuger (7) durch Sublimation niedergeschlagenen und angesammelten Desinfektionsmittels (3) veranlaßt, bevor diese Schicht eine Stärke aufweist, welche die Arbeitsweise des Kälteerzeugers (7) beeinträchtigen kann, und daß eine Abführeinrichtung (10) das getaute Desinfektionsmittel (3) aus der Kryostatkammer (2) ableitet,
**dadurch gekennzeichnet**,
die Steuerung (9) zur Durchführung des Verfahrens nach Anspruch 1 ausgestaltet ist.

17. Vorrichtung nach Anspruch 16,
**dadurch gekennzeichnet,**
**daß** die Vernebelungsvorrichtung (8') mittels Ultraschall die Feinheit des Nebels erzeugt.

18. Vorrichtung nach Anspruch 16 oder 17,
**dadurch gekennzeichnet,**
**daß** die Einrichtung (8) zur Bereitstellung und Einbringung eines Desinfektionsmittels (3) einen Ventilator (14) aufweist, der die Kryostatkammer (2) von oben mit dem vernebelten Desinfektionsmittel (3) beaufschlagt.

19. Vorrichtung nach Anspruch 16, 17 oder 18,
**dadurch gekennzeichnet,**
**daß** der Kälteerzeuger (7) zur Bewirkung eines schnelleren Abtauens eine Heizeinrichtung (11) aufweist.

20. Vorrichtung nach einem der Ansprüche 16 bis 19,
**dadurch gekennzeichnet,**
**daß** der Kälteerzeuger (7) eine Auffangvorrichtung (13) für die Zuführung des abgetauten Desinfektionsmittels (3) zu der Abführvorrichtung (10) aufweist.

21. Vorrichtung nach einem der Ansprüche 16 bis 20,
**dadurch gekennzeichnet,**
**daß** sie eine Eingabevorrichtung (12) zur manuellen Ingangsetzung der Desinfektionsmitteleinführung (3') aufweist.

22. Vorrichtung nach einem der Ansprüche 16 bis 21,
**dadurch gekennzeichnet,**
**daß** die Steuerung (9) derart ausgebildet ist, daß mindestens eine Desinfektionsmitteleinführung (3') täglich bewirkt wird.

23. Vorrichtung nach einem der Ansprüche 16 bis 22,
**dadurch gekennzeichnet,**
**daß** die Steuerung (9) derart ausgebildet ist, daß sie das Abtauen des Kälteerzeugers (7) einmal täglich bewirkt.

24. Vorrichtung nach einem der Ansprüche 16 bis 23,
**dadurch gekennzeichnet,**
**daß** eine Einrichtung zur beschleunigten Entfernung der reifartigen Ablagerung eines Desinfektionsmittels (3) am Mikrotommesser (5) nach der notwendigen Einwirkzeit vorgesehen ist.

25. Vorrichtung nach Anspruch 24,
**dadurch gekennzeichnet,**
**daß** die Einrichtung zur beschleunigten Entfernung eine Einrichtung zur mechanischen Entfernung der reifartigen Ablagerung ist.

26. Vorrichtung nach Anspruch 24,
**dadurch gekennzeichnet,**
**daß** die Einrichtung zur beschleunigten Entfernung eine Heizung für das Mikrotommesser (5) ist.

27. Vorrichtung nach Anspruch 26,
**dadurch gekennzeichnet,**
**daß** die Einrichtung zur beschleunigten Entfernung auch eine Kühleinrichtung aufweist, die das Mikrotommesser (5) nach der Entfernung der reifartigen Ablagerung auch wieder kühlt.

28. Vorrichtung nach Anspruch 26 und 27,
**dadurch gekennzeichnet,**
**daß** die Kühleinrichtung ein Peltierelement (15) ist.

29. Vorrichtung nach einem der Ansprüche 16 bis 28,
**dadurch gekennzeichnet,**
**daß** sie für die Handhabung einer Wasserstoffperoxidlösung als Desinfektionsmittel (3) optimiert ist.

## Claims

1. Method for disinfecting a microtome cryostat (1) comprising
a) introduction of a disinfection agent (3) into the cryostat chamber (2) at a temperature below 0°C, wherein the disinfection agent (3) is effective at said temperature,
b) action of the disinfection agent (3) on the cryostat chamber (2),
c) disposal of the disinfection agent (3) by deposit in a colder area (6) and removal therefrom
**characterized by** the following embodiment of said method steps:
a) said disinfection agent (3) is introduced as a mist,
b) said disinfection agent (3) is effective as a frost deposit in said cryostat chamber (2),
c) said frost deposit being displaced into a colder area (6) by sublimation is converted to liquid by sporadic defrosting phases of said area (6) and thereby removed.

2. Method of claim 1, **characterized in that** the disinfection agent (3) is introduced into the cryostat chamber (2) at a normal operating temperature thereof.

3. Method of claim 1 or 2, **characterized in that** the introduction of the nebulized disinfection agent (3) takes 3 minutes.

4. Method of claim 1, 2 or 3, **characterized in that** 15 to 25 ml of disinfection agent (3) are introduced into the cryostat chamber (2).

5. Method according to one of the claims 1 to 4, **characterized in that** the nebulization of the disinfection agent (3) is achieved by means of ultrasound before introduction (3') into the cryostat chamber (2).

6. Method according to one of the claims 1 to 5, **characterized in** a cooling element (7) is used as the colder area (6), which is heated at sporadic intervals for thawing and draining away of the disinfection agent (3) precipitated there such that a frozen disinfection agent layer cannot develop to such a depth as to impair function of the cooling element (7).

7. Method of claim 6, **characterized in that** thawing and draining away is performed once per day.

8. Method according to one of the claims 1 to 7, **characterized in that** introduction of disinfection agent (3') is performed when disinfection is required.

9. Method of claim 8, **characterized in that** introduction of the disinfection agent (3') is performed automatically at least once per day.

10. Method according one of the claims 1 to 9, **characterized in that** the microtome (4) is restarted when the frost deposit of disinfection agent on the microtome blade (5) has been removed.

11. Method according to claim 10, **characterized in that** removal of the frost deposit of the disinfection agent (3) on the microtome blade (5) after a necessary disinfecting time is performed mechanically in order to restart the microtome (4) more quickly after disinfection.

12. Method according to of claim 10, **characterized in that** removal of the disinfection agent layer from the microtome blade (5) after a necessary disinfecting time is accelerated by heating up the blade.

13. Method according to claim 12, **characterized in that** return to an operating temperature of the microtome blade (5) is accelerated by cooling the blade.

14. Method according to one of the claims 1 to 13, **characterized in that** a hydrogen peroxide solution is used as the disinfection agent (3).

15. Method according to claim 14, **characterized in that** the solution contains a proportion of silver.

16. Device for disinfecting a microtome cryostat (1) by a method according one of the above mentioned claims having a microtome (4), a cooling element (7), a device (8) for providing and introducing a disinfection agent (3) into the cryostat chamber (2) and a control device (9),
wherein said device (8) for providing and introducing a disinfection agent (3) is such a nebulization device (8') that a frost deposit of said disinfection agent (3) into said cryostate chamber (2) in the cryosate chamber (3) is possible by fineness and type of introduction the nebulized disinfection agent (3) into said cryostate chamber (2), wherein the control device is configured to trigger defrosting of the disinfection agent (3) being deposited and collected on the cooling element (7) by sublimation, before the layer reaches a thickness that would impair function of said cooling element (7), and wherein a drain device (10) removes the thawed disinfection agent (3) from the cryostate chamber (2), **characterized in that** the control device (9) is provided for carrying out the method of claim 1.

17. Device according to claim 16, **characterized in that** said nebulization element (8') produces fineness of the mist by means of ultrasound.

18. Device according to claim 16 or 17, **characterized in that** said device (8) for providing and introducing a disinfection agent (3) comprises a ventilator (14) that applies nebulized disinfection agent (3) into the cryostat chamber (2) from above.

19. Device according to claim 16, 17 or 18, **characterized in that** said cooling element (7) comprises a heating element (11) to accelerate thawing.

20. Device according to one of the claims 16 to 19, **characterized in that** said cooling element (7) has a collection element (13) for guiding thawed disinfection agent (3) to said drain device (10).

21. Device according to one of the claims 16 to 20, **characterized in that** it comprises an input unit (12) for manually starting introduction of the disinfection agent (3').

22. Device according to one of the claims 16 to 21, **characterized in that** the control (9) is constituted in such a way that introduction of the disinfection agent (3') is triggered at least once per day.

23. Device according to one of the claims 16 to 22, **characterized in that** the control (9) is constituted to trigger thawing of said cooling element (7) once per day.

24. Device according to one of the claims 16 to 23, **characterized in that** a device is provided for accelerated removal of the frost deposit of disinfection agent (3) on the microtome blade (5) after a necessary disinfecting time.

25. Device according to claim 24, **characterized in that** said device for accelerated removal is a device for mechanical removal of the frost deposit.

26. Device according to claim 24, **characterized in that** said device for accelerated removal is a heating device for the microtome blade (5).

27. Device according to claim 26, **characterized in that** said device for accelerated removal further comprises a cooling device that cools the microtome blade (5) after removal of the frost deposit.

28. Device according to claim 26 and 27, **characterized in that** said cooling device comprises a Peltier device (15) .

29. Device according to one of the claims 16 to 28, **characterized in that** it is optimized for handling of a hydrogen peroxide solution as the disinfection agent (3) .

## Revendications

1. Procédé de désinfection d'un cryostat à microtome (I) avec :
a) application d'un moyen de désinfection (3) dans la chambre cryostatique (2) à moins de 0 °C, le moyen de désinfection (3) étant actif à cette température ;
b) action du moyen de désinfection (3) sur la chambre cryostatique (2) ;
c) enlèvement du moyen de désinfection (3) par descente dans une zone plus froide (6) et évacuation depuis cet endroit ;
**caractérisé par** la configuration suivante de ces étapes de procédé :
a) le moyen de désinfection (3) est appliqué sous forme nébulisée ;
b) le moyen de désinfection (3) agit dans la chambre cryostatique (2) sous la forme d'un dépôt givré ;
c) le dépôt de type givré déposé par sublimation est fluidifié dans une région plus froide (6) par des phases de dégel sporadique de cette région (6) et ainsi évacué.

2. Procédé selon la revendication 1, **caractérisé en ce que** le moyen de désinfection (3) est délayé dans la chambre cryostatique (2) à la température de fonctionnement habituelle de ladite chambre.

3. Procédé selon la revendication 1 ou 2, **caractérisé en ce que** l'application du moyen de désinfection (3) nébulisé dure 3 minutes.

4. Procédé selon la revendication 1, 2 ou 3, **caractérisé en ce que** 15 à 25 ml de moyen de désinfection (3) sont amenés dans la chambre cryostatique (2).

5. Procédé selon l'une quelconque des revendications 1 à 4, **caractérisé en ce que** la nébulisation du moyen de désinfection (3) est réalisée au moyen d'ultrasons avant l'introduction (3') dans la chambre cryostatique (2).

6. Procédé selon l'une quelconque des revendications 1 à 5, **caractérisé en ce que** la région plus froide (6) sert de générateur de froid (7) réchauffé, pour dégeler et évacuer le moyen de désinfection (3) y étant stocké, à intervalles sporadiques tels qu'aucune couche de moyen de désinfection gelée ne peut se former à une épaisseur entravant la capacité fonctionnelle du générateur de froid (7).

7. Procédé selon la revendication 6, **caractérisé en ce que** le dégel et l'évacuation sont effectués une fois par jour.

8. Procédé selon l'une quelconque des revendications 1 à 7, **caractérisé en ce que** la mise en route d'introduction de moyen de désinfection (3') se produit en fonction du besoin de désinfection respectif.

9. Procédé selon la revendication 8, **caractérisé en ce qu'**au moins une introduction de moyen de désinfection (3') se produit automatiquement quotidiennement.

10. Procédé selon l'une quelconque des revendications 1 à 9, **caractérisé en ce que** le microtome (4) est remis en fonctionnement lorsque la couche de moyen de désinfection givrée placée sur le microtomètre (5) est enlevée.

11. Procédé selon la revendication 10, **caractérisé en ce que** l'enlèvement du dépôt givré du moyen de désinfection (3) se produit de façon mécanique au niveau du microtomètre (5) après écoulement du temps d'action nécessaire pour permettre une remise en fonctionnement plus rapide du microtome (4) après une désinfection.

12. Procédé selon la revendication 10, **caractérisé en ce que** le retrait de la couche de moyen de désinfection sur le microtomètre (5) après écoulement du temps d'action nécessaire est accéléré par un réchauffement de celui-ci.

13. Procédé selon la revendication 12, **caractérisé en ce que** la réatteinte de la température de fonctionnement du microtomètre (5) est accélérée par un refroidissement de celui-ci.

14. Procédé selon l'une quelconque des revendications 1 à 13, **caractérisé en ce que** le moyen de désinfection (3) utilisé est une solution de peroxyde d'hydrogène.

15. Procédé selon la revendication 14, **caractérisé en ce que** ladite solution contient une certaine teneur en argent.

16. Dispositif de désinfection d'un cryostat à microtome (1) suivant un procédé selon l'une quelconque des revendications précédentes, avec un microtome (4), un générateur de froid (7), un dispositif (8) de mise à disposition et d'application d'un moyen de désinfection (3) dans la chambre cryostatique (2) ainsi qu'un élément de commande (9) ;
le dispositif (8) de mise à disposition et d'application d'un moyen de désinfection (3) étant un dispositif de nébulisation (8') configuré de telle sorte qu'un dépôt givré du moyen de désinfection (3) dans la chambre cryostatique (2) est activé par capillarité et par type d'application du moyen de désinfection (3) nébulisé dans la chambre cryostatique (2), l'élément de commande (9) étant réalisé de telle sorte qu'il déclenche un dégel du moyen de désinfection (3) descendu et accumulé au niveau du générateur de froid (7) par sublimation avant que l'épaisseur de cette couche ne puisse entraver le bon fonctionnement du générateur de froid (7) et un dispositif d'évacuation (10) évacuant le moyen de désinfection (3) dégelé hors de la chambre cryostatique (2) ;
**caractérisé en ce que** l'élément de commande (9) est configuré pour mettre en oeuvre le procédé selon la revendication 1.

17. Dispositif selon la revendication 16, **caractérisé en ce que** le dispositif de nébulisation (8') régule la capillarité de la nébulisation par le biais d'ultrasons.

18. Dispositif selon la revendication 16 ou 17, **caractérisé en ce que** le dispositif (8) de mise à disposition et d'application d'un moyen de désinfection (3) comporte un ventilateur (14) alimentant la chambre cryostatique (2) en moyen de désinfection (3) nébulisé par en haut.

19. Dispositif selon la revendication 16, 17 ou 18, **caractérisé en ce que** le générateur de froid (7) comporte un dispositif de chauffage (11) pour permettre un dégel plus rapide.

20. Dispositif selon l'une quelconque des revendications 16 à 19, **caractérisé en ce que** le générateur de froid (7) comporte un dispositif de collecte (13) permettant d'amener le moyen de désinfection (3) dégelé au dispositif d'évacuation (10).

21. Dispositif selon l'une quelconque des revendications 16 à 20, **caractérisé en ce qu'**il comporte un dispositif de saisie (12) permettant de réaliser une mise en route manuelle d'introduction de moyen de désinfection (3').

22. Dispositif selon l'une quelconque des revendications 16 à 21, **caractérisé en ce que** l'élément de commande (9) est réalisé de telle sorte qu'au moins une introduction de moyen de désinfection (3') est opérée quotidiennement.

23. Dispositif selon l'une quelconque des revendications 16 à 22, **caractérisé en ce que** l'élément de commande (9) est réalisé de telle sorte qu'il opère le dégel du générateur de froid (7) une fois par jour.

24. Dispositif selon l'une quelconque des revendications 16 à 23, **caractérisé en ce qu'**un dispositif de retrait accéléré du dépôt givré d'un moyen de désinfection (3) au niveau du microtomètre (5) est prévu après écoulement du temps d'action nécessaire.

25. Dispositif selon la revendication 24, **caractérisé en ce que** le dispositif de retrait accéléré est un dispositif de retrait mécanique du dépôt givré.

26. Dispositif selon la revendication 24, **caractérisé en ce que** le dispositif de retrait accéléré est un chauffage prévu pour le microtomètre (5).

27. Dispositif selon la revendication 26, **caractérisé en ce que** le dispositif de retrait accéléré comporte également un dispositif de refroidissement refroidissant de nouveau le microtomètre (5) après enlèvement du dépôt givré.

28. Dispositif selon la revendication 26 et 27, **caractérisé en ce que** le dispositif de refroidissement est un élément de Peltier (15).

29. Dispositif selon l'une quelconque des revendications 16 à 28, **caractérisé en ce qu'**il est optimisé pour la manipulation d'une solution de peroxyde d'hydrogène servant de moyen de désinfection (3).
